# EUROPEAN PATENT SPECIFICATION

(11) **EP 1 044 607 B1**
(45) Date of publication and mention of the grant of the patent: **18.02.2004**
(21) Application number: 00107774.2
(22) Date of filing: 11.04.2000
(51) Int. Cl.: A01M 1/20

(54) **Electric heating device for the vaporisation of deodorants or insecticides**
Elektrisches Heizelement für die Verdunstung von Insektizide oder Parfüms
Element électrique de chauffage pour la vaporisation des insecticides ou parfums

(30) Priority: 13.04.1999 IT MI990758
(43) Date of publication of application: 18.10.2000
(73) Proprietor: ZOBELE HOLDING S.P.A., 38100 TRENTO (IT)
(72) Inventor: Ambrosi, Stefano, 38014 Gardolo (IT); Pedrotti, Andrea, 30070 Pietramurata (IT); Zobele, Franco, 38100 Trento (IT)
(74) Representative: Faggioni, Marco, Dr. Ing.

(56) References cited:
- WO-A-97/02054
- DE-A- 4 301 912
- FR-A- 2 523 861
- US-A- 4 391 781

## Description

The present invention concerns an electric heater for the vaporization of deodorant or insecticide substances and, in particular, a heater. wherein the substance to be vaporized is held, as a gel, into a rectangular container, or is soaked in a liquid state into a solid support plate of porous material, normally also having a rectangular shape.

A problem of this type of heater consists in the uniform supply of heat over the whole surface of the container or plate, so as to ensure a homogeneous and complete evaporation of the active substance and prevent, furthermore, localized overheating which could cause the degradation of said substance.

In the heaters known up-to-date in the prior art, this result has been obtained with two different solutions, according to the type of heating element used. In a first solution, a linear heating element - for instance a conventional wire or sheet-metal resistance onto a cylindrical support - or a circular heating element - for instance a PTC posistor - is associated, to or a possibly embedded into a ceramic or metallic diffusion element allowing the heat to be homogeneously, or almost homogeneously, distributed over the whole rectangular surface of the WO-A-97/02054 discloses an heater wherein the heating device is associated to a ceramic element container or plate./In a second solution the heating element is in the form of a flexible wire resistance which embedded, by molding, into a strip of plastic material, of shape and size similar to those of the container or plate to be heated. The wire resistance is positioned into the strip in a loop pattern, apt to cover said strip in a sufficiently uniform way and thereby guarantee the required homogeneity in supplying the heat to the active substance.

Nevertheless, both the aforedescribed technical solutions have the drawback of involving high costs, determined both by the actual costs of the materials used - the metallic or ceramic diffusion elements, in the first case, and the flexible wire resistance in the second case - and by the production and assembly of the heater which involve a more complex and costly process. Thus, the object of the present invention is to provide a heater of the type described heretofore which, while making use of a linear or circular heating element which is not combined with any type of diffusion elements, allows to obtain a sufficiently homogeneous heat distribution over the entire surface of the rectangular container or plate holding the active substance.

According to the present invention, said object is reached by means of an electric heater for the vaporization of deodorant or insecticide substances held into supports, generally of rectangular shape, the heater comprising a linear or circular heating element and a wall parting the heating element from the support for the substance to be vaporized, characterized in that said parting wall has a thickness which varies in a manner directly proportional to the heat flowing through the same.

The present invention will now be described in further detail, with reference to some preferred embodiments thereof, illustrated on the accompanying drawings, in which:
Fig. 1 is a side elevation view of a first embodiment, with single cartridge, of the electric heater according to the present invention;
Fig. 2 is a section view, on an enlarged scale, of the heater shown in fig. 1;
Fig. 3 is a front elevation view of the inner side of the half-shell of the heater of fig. 1, carrying the heating element and the plug;
Fig. 4 is a front elevation view of the inner side of the half-shell of the heater of fig. 1, carrying the cartridge of the substance to be vaporized;
Fig. 5 is a section view along the line V-V of fig. 4;
Fig. 6 is a front elevation view of the inner side of one of the half-shells of a second embodiment, with double cartridge, of the electric heater according to the present invention; and
Fig. 7 is a section view along the line VII-VII of fig. 6, wherein, for better clearness, the plug is not shown.

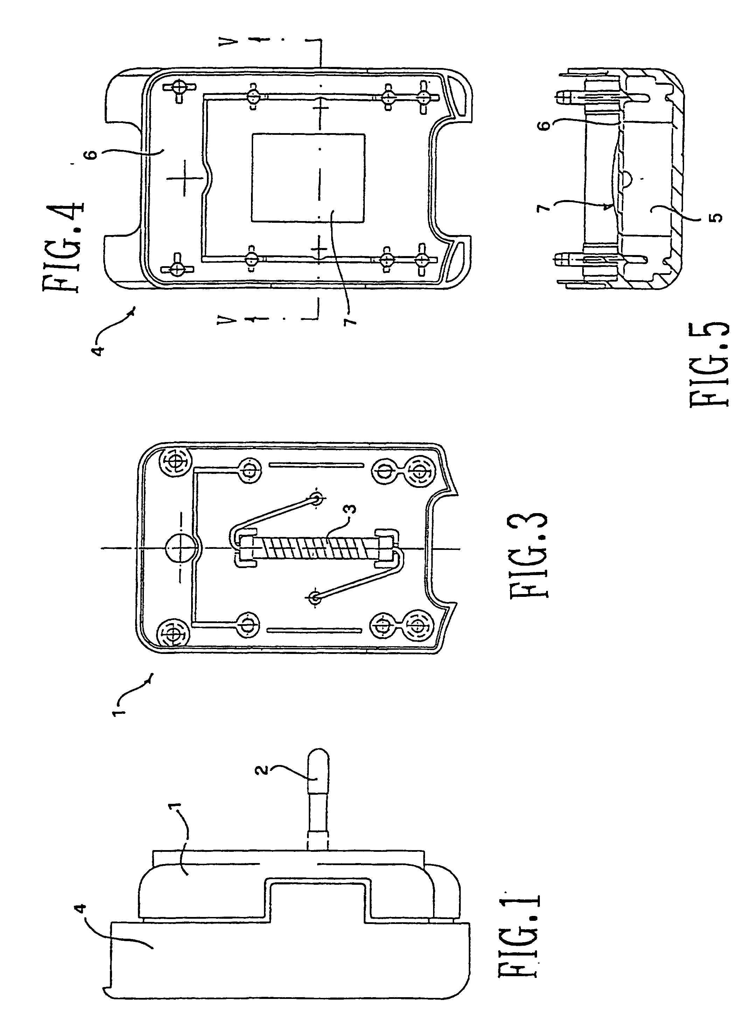

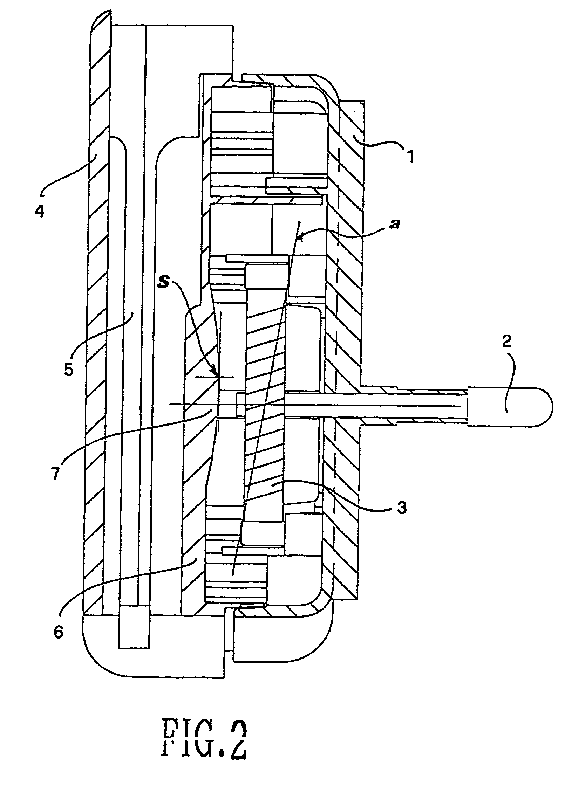

The electric heater according to the present invention consists - in per se known manner - of two half-shells, formed by injection molding of a suitable plastic material and apt to be coupled together by snapping, by screws, or other known systems. In the first embodiment of the heater - illustrated in figs. 1 to 5 - the half-shell 1 comprises a plug 2 (fig. 1) and a heating element 3 in the form of a cylindrical coil resistor, whose terminals are directly inserted into the pins of the plug 2 (fig. 3).

The other half-shell 4 of the heater (figs. 4 and 5) comprises the housing 5 for the cartridge of the deodorant or insecticide substance, and a wall 6 which parts said cartridge from the heating element 3.

According to the invention, the wall 6 - which is integrally formed with the remaining part of the half-shell 4, for instance by injection molding - instead of having a constant thickness as in the known-type electric heaters, comprises a central portion (approximately indicated by the reference number 7) of increased and variable thickness. In particular - as clearly illustrated in the section views of figs. 2 and 5 - said portion comprises a swell, both lengthwise and crosswise, so as to be thicker in correspondence of the heating element 3 and thereby provide a higher resistance to the heat flowing through, just where the heat flow is stronger. For this purpose, while the point of maximum thickness of the portion 7, in the transversal direction, coincides with the central part of the heater (fig. 5), and thus with the axis of the heating element 3, the point of maximum thickness in the longitudinal direction - indicated by the arrow S in fig. 2 - is slightly higher in respect of the middle point of the heating element 3 (coinciding with the axis of the pins of the plug 2), so as to take into account the upward convective component of the heat flow produced by the resistor.

To simplify the drawing, the portion 7 of increased thickness has been given a rectangular shape, but it is evident that different shapes can be given to said portion 7, so as to make its thickness proportional to the heat actually flowing through the same; the result is a homogeneous or substantially homogeneous heat flow, beyond the wall 6, into the housing 5 holding the cartridge of the substance to be vaporized. Likewise, the central portion 7 may be given, for example, an ellipsoidal shape, or it may be shaped as an isosceles trapezium, with the shorter side positioned in the lower half of the heater, so as to more efficiently compensate the convective effect of the heat flow produced by the resistor 3. The maximum rate of thickness variation can be adjusted according to the characteristics of conductivity of the plastic material with high thermal resistance used to form the wall 6, and to the characteristics of the heat flow produced by the resistor 3.

The heat flow compensation - always in order to make as homogeneous as possible the heat flow which reaches the cartridge of the substance to be vaporized, in the housing 5 - can finally be operated also by varying the inclination of the resistor 3 on a vertical plane, so that its upper end may be positioned at a higher distance from the wall 6 than its lower end, as schematically indicated by the dashed line a in fig. 2.

The technical solution supplied by the present invention can of course be applied to a wide variety of electric heaters and it allows, in a simple and economic manner, to reach excellent results of homogenization of the heat flow onto the cartridge, also with a circular or linear heating element, or anyhow with a heating element of smaller dimensions than those of the cartridge to be heated. Since, in fact, the thickness is increased in the same wall which forms one of the two half-shells of the electric heater, the only modification required is that which should be introduced in the mold of said half-shell during the planning step; this allows to totally eliminate from the production of the heater, both the supply of additional ceramic or metallic heat diffusion materials, and the mounting thereof in the electric heater.

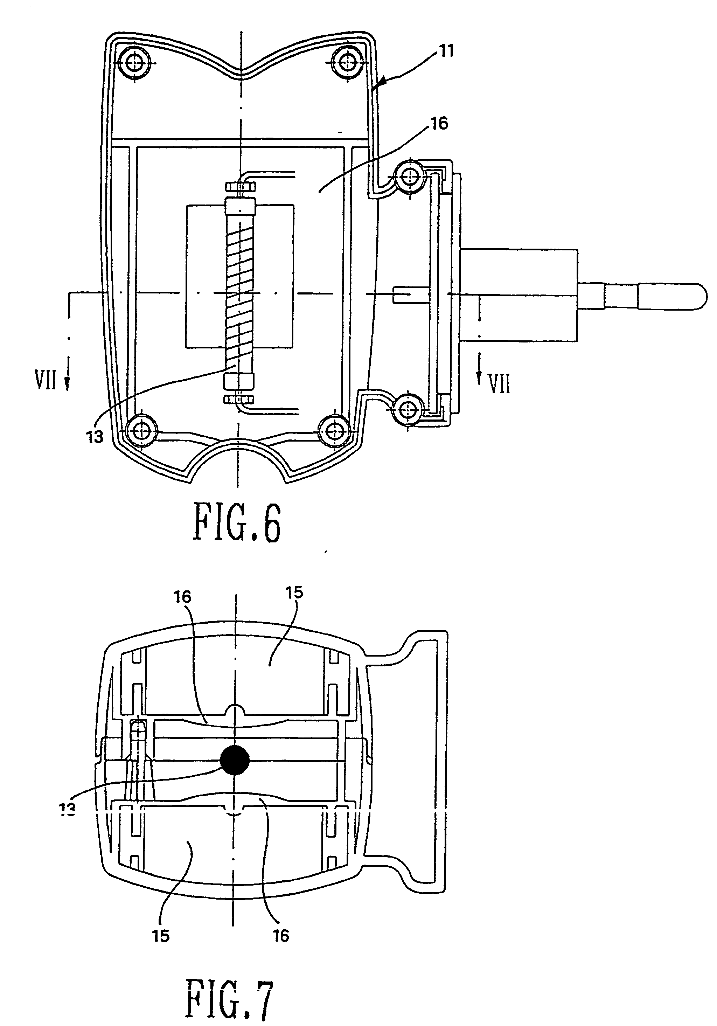

According to a further embodiment of the present invention illustrated by mere way of non-limiting example in figs. 6 and 7 - the electric heater can be foreseen to house a double cartridge. In said heater, a heating element 13 is housed into one of the half-shells 11 which form the heater, each of said half-shells having a respective wall 16 which parts the heating element 13 from the two housings 15 for the cartridges of the substance to be vaporized. The thickness variations of the walls 16 will of course be planned on the basis of the same characteristics reported further above.

As it can be understood from the previous description, the present invention can be adopted in a great number of different variants, all within reach of a person skilled in the art and all falling within the scope of the present invention, such as defined in the following claims.

## Claims

1. Electric heater for the vaporisation of deodorants or insecticides contained in rectangular supports, said heater comprising at least a linear or circular heating device (3, 13) and at least a wall (6, 16) which parts the heating device from the support (5) of the substance to be vaporised, **characterised in that** the thickness of said parting wall (6, 16) is greater in correspondence of said heating device (3, 13), thereby providing a higher resistance to the heat flowing through the parting wall (6, 16) were the heat flow is stronger.

2. Electric heater as in claim 1), wherein said parting wall (6, 16) is different in thickness in a way directly proportional to the heat flow which passes therethrough.

3. Electric heater as in any one of the previous claims, wherein said heating device (3, 13) is formed by a vertically positioned cylindrical resistance.

4. Electric heater as in claim 3), wherein any change in thickness of said parting wall (6, 16) along a direction perpendicular to the axis of said resistance (3, 13) is symmetrical to said axis.

5. Electric heater as in claim 3), wherein the change in thickness of said parting wall (6, 16), along a direction parallel to the axis of said resistance (3, 13), shows a maximum moved towards the top in respect of the middle point of said resistance.

6. Electric heater as in claim 3), wherein the area with a variable thickness of said parting wall (6, 16) which is different in thickness has a rectangular shape, a trapezoidal shape with the shorter side positioned in the lower half of the heater, or an ellipsoidal shape.

7. Electric heater as in claim 3), wherein the longitudinal axis (9) of the electrical resistance is inclined as to a vertical line, so that the upper end of the resistance has a longer distance from the parting wall (6, 16) than the lower end thereof.

8. Electric heater as in any one of the previous claims, wherein said parting wall (6, 16) is integral with the half shell (4) of the heater which comprises the same and is made of a plastic material with a high heat resistance.

## Patentansprüche

1. Elektrisches Heizgerät für die Verdunstung von in rechteckigen Trägem enthaltenen Deodorants beziehungsweise Insektiziden, wobei genanntes Heizgerät mindestens ein lineares oder kreisförmiges Heizelement (3, 13) und mindestens eine Wand (6, 16) umfaßt, die das Heizelement von dem Träger (5) der zu verdunstenden Substanz trennt, **dadurch gekennzeichnet, daß** die Dicke genannter Trennwand größer in Übereinstimmung mit genanntem Heizelement (3, 13) ist, wodurch ein höherer Widerstand gegen die durch die Trennwand (6, 16) strömende Wärme bereitgestellt wird, wo der Wärmestrom stärker ist.

2. Elektrisches Heizgerät nach Anspruch 1, **dadurch gekennzeichnet, daß** genannte Trennwand (6, 16) in einer zum Wärmestrom, der dort hindurchtritt, direkt proportionalen Weise unterschiedlich dick ist.

3. Elektrisches Heizgerät nach einem der vorangehenden Ansprüche, **dadurch gekennzeichnet, daß** genanntes Heizelement (3, 13) von einem vertikal positionierten zylindrischen Widerstand gebildet ist.

4. Elektrisches Heizgerät nach Anspruch 3, **dadurch gekennzeichnet, daß** eine Dickenänderung der Trennwand (6, 16) entlang einer zur Achse genannten Widerstands (3, 13) senkrechten Richtung symmetrisch zu genannter Achse ist.

5. Elektrisches Heizgerät nach Anspruch 3, **dadurch gekennzeichnet, daß** die Dickenänderung der Trennwand (6, 16) entlang einer zur Achse genannten Widerstands (3, 13) parallelen Richtung ein Maximum aufweist, das in Richtung zur Oberseite in Bezug auf den Mittelpunkt genannten Widerstands verschoben ist.

6. Elektrisches Heizgerät nach Anspruch 3, **dadurch gekennzeichnet, daß** das Gebiet mit einer variablen Dicke genannter Trennwand (6, 16), die eine unterschiedliche Dicke aufweist, eine rechteckige Gestalt, eine Trapezgestalt, wobei die kürzere Seite in der unteren Hälfte des Heizgerätes positioniert ist, oder eine ellipsenförmige Gestalt aufweist.

7. Elektrisches Heizgerät nach Anspruch 3, **dadurch gekennzeichnet, daß** die Längsachse (9) des elektrische Widerstandes zu einer vertikalen Linie geneigt ist, so daß das obere Ende des Widerstands eine längere Entfernung von der Trennwand (6, 16) als dessen unteres Ende aufweist.

8. Elektrisches Heizgerät nach einem der vorangehenden Ansprüche, **dadurch gekennzeichnet, daß** genannte Trennwand (6, 16) einteilig mit der Halbschale (4) des Heizgerätes, das selbige umfaßt, ist und aus einem Kunststoffmaterial mit einer hohen Wärmebeständigkeit hergestellt ist.

## Revendications

1. Dispositif de chauffage électrique destiné à la vaporisation de désodorisants ou d'insecticides contenus dans des supports rectangulaires, ledit dispositif de chauffage comprenant au moins un dispositif de chauffage linéaire ou circulaire (3, 13) et au moins une paroi (6, 16) qui sépare le dispositif de chauffage du support (5) de la substance à vaporiser, **caractérisé en ce que** l'épaisseur de ladite paroi de séparation (6, 16) est plus grande en correspondance avec ledit dispositif de chauffage (3, 13), offrant ainsi une résistance plus élevée à la chaleur circulant à travers la paroi de séparation (6, 16) où le flux de chaleur est plus fort.

2. Dispositif de chauffage électrique selon la revendication 1), dans lequel ladite paroi de séparation (6, 16) présente une épaisseur différente d'une façon directement proportionnelle au flux de chaleur qui la traverse.

3. Dispositif de chauffage électrique selon l'une quelconque des revendications précédentes, dans lequel ledit dispositif de chauffage (3, 13) est constitué d'une résistance cylindrique positionnée verticalement.

4. Dispositif de chauffage électrique selon la revendication 3), dans lequel toute variation d'épaisseur de ladite paroi de séparation (6, 16) le long d'une direction perpendiculaire à l'axe de ladite résistance (3, 13) est symétrique audit axe.

5. Dispositif de chauffage électrique selon la revendication 3), dans lequel la variation d'épaisseur de ladite paroi de séparation (6, 16), le long d'une direction parallèle à l'axe de ladite résistance (3, 13), présente un maximum déplacé vers le haut par rapport au point milieu de ladite résistance.

6. Dispositif de chauffage électrique selon la revendication 3), dans lequel la zone présentant une épaisseur variable de ladite paroi de séparation (6, 16) qui est différente en épaisseur, présente une forme rectangulaire, une forme trapézoïdale, le côté plus court étant positionné dans la moitié inférieure du dispositif de chauffage, ou une forme ellipsoïdale.

7. Dispositif de chauffage électrique selon la revendication 3), dans lequel l'axe longitudinal (9) de la résistance électrique est incliné par rapport à une ligne verticale, de sorte que l'extrémité supérieure de la résistance présente une dimension plus longue par rapport à la paroi de séparation (6, 16) que son extrémité inférieure.

8. Dispositif de chauffage électrique selon l'une quelconque des revendications précédentes, dans lequel ladite paroi de séparation (6, 16) fait partie intégrante de la demi-coque (4) du dispositif de chauffage qui comprend celle-ci et est constituée d'une matière plastique présentant une résistance à la chaleur élevée.
